# EUROPEAN PATENT SPECIFICATION

(11) **EP 0 999 198 B1**
(45) Date of publication and mention of the grant of the patent: **09.06.2004**
(21) Application number: 99308575.2
(22) Date of filing: 28.10.1999
(51) Int. Cl.: C07C 51/12, C07C 53/08

(54) **Process for the production of acetic acid**
Verfahren zur Herstellung von Essigsäure
Procédé pour la préparation de l'acide acétique

(30) Priority: 03.11.1998 GB 9824011
(43) Date of publication of application: 10.05.2000
(73) Proprietor: BP Chemicals Limited, London EC2M 7BA (GB)
(72) Inventor: Clode, Kirsten Everald, Hull HU12 8DS (GB); Muskett, Michael James, Hull HU12 8DS (GB)
(74) Representative: Perkins, Nicholas David

(56) References cited:
- EP-A- 0 752 406
- WO-A-98/22420

## Description

The present invention relates to a process for the production of acetic acid, and in particular for the production of acetic acid by the carbonylation of methanol and/or a reactive derivative thereof in the presence of an iridium catalyst

Acetic acid is a well known commodity chemical which has many industrial uses. Processes for the production of acetic acid are carried out by carbonylating an alcohol in the presence of a Group VIII metal carbonylation catalyst, a halogen-containing co-catalyst and a finite concentration of water. The catalyst used in the process may be a rhodium or an iridium-containing catalyst

Carbonylation process for the production of carboxylic acid in the presence of a rhodium containing catalyst is described in, for example, US-A-3769329; GB-A-1468940; GB-A-1538783 EP-A-0087870.

Carbonylation processes for the production of carboxylic acid in the presence of an iridium-containing catalyst is described in, for example, GB-A-1234641; US-A-3772380; DE-A-1767150; EP-A-0616997; EP-A-0618184; EP-A-0618183, EP-A-0657386, EP 0 752 406 and WO 98/22420.

The general catalysed carbonylation process is described in an article by Howard et al in Catalysis Today, 18 (1993), 325-354. The continuous catalysed, homogeneous methanol carbonylation process is said to consist of three basic sections; reaction, purification and off-gas treatment. The reaction section comprises a stirred tank reactor, operated at elevated temperature, and a flash vessel. Liquid reaction composition is withdrawn from the reactor and is passed through a flashing valve to the flash tank where the majority of the lighter components of the liquid composition (methyl iodide, methyl acetate and water) together with the product acetic acid are vapourised. The vapour fraction is then passed to the purification section whilst the liquid fraction (comprising the catalyst in acetic acid) is recycled to the reactor. The purification section is said to comprise a first distillation column (the light ends column), a second distillation column (the drying column) and a third distillation column (the heavy ends column). In the light ends column, methyl iodide and methyl acetate are removed overhead along with some water and acetic acid. The vapour is condensed and allowed to separate into two phases into a decanter, both phases being returned to the reactor. Wet acetic acid is removed from the light ends column as a side draw and is fed to the drying column where water is removed overhead and an essentially dry acetic acid stream is removed from the base of the distillation zone. The overhead water stream from the drying column is recycled to the reaction section. Heavy liquid by-products are removed from the base of the heavy ends column with the product acetic acid being taken as a side stream.

In the carbonylation process, particularly the carbonylation of methanol to acetic acid, carbonylation takes place in the presence of a finite concentration of water. Typically, the concentration of water is maintained at a low level, for example less than 14 wt%, preferably less than 8wt%. The carbonylation process is a continuous process and the level of reactants are also maintained at a constant level. In order to maintain the inventory of the liquid reaction composition in the reactor of a rhodium-catalysed carbonylation process, a recycle flow from the head of the drying column is varied. A problem, however, associated with this act is that in varying the recycle flow to maintain the liquid reaction composition inventory, the balance of the water concentration may also be altered. In general, a change in water concentration does not present a serious problem in the rhodium-catalysed carbonylation process, since water does not affect the rate of reaction. In the iridium-catalysed system the rate of reaction is affected by the water content in the reactor; there being an optimum amount of water for maximum rates on a rate curve with adverse effects on rate with more/less water in the reactor. When using the standard rhodium techniques for reaction composition inventory control, the plant water balance was easily upset by any change in a recycle from the heads of the second (drying) distillation column, which is the main stream used for reaction composition inventory control. These upsets in water balance can cause serious operational problems to the point where reactor feed rates would have to be significantly reduced.

EP-A-0752406 relates to a process for the production of acetic acid by carbonylation in the presence of an iridium catalyst and methyl iodide co-catalyst. In the continuous experiments water is recycled from the overhead of the drying column to the reactor.

WO 98/22420 relates to the production of acetic acid by carbonylation of methanol and/or a reactive derivative thereof wherein the water concentration in the liquid reaction composition is maintained at a steady-state concentration by inter-alia recovering and disposing of water from at least part of the overhead process stream from the light ends column.

To overcome this problem associated with the iridium-catalysed carbonylation process, we have found that the inventory of liquid reaction composition in the carbonylation reactor may be maintained without adversely affecting the water balance, and ultimately the rate of reaction, by introducing an alternative recycle stream into the reactor.

Accordingly, the present invention provides a process for the production of acetic acid which comprises the steps of :
a) feeding methanol and/or a reactive derivative thereof and carbon monoxide to a carbonylation reactor in which there is maintained during the course of the process a liquid reaction composition comprising an iridium catalyst, methyl iodide co-catalyst, a finite amount of water, methyl acetate and optionally one or more promoters,
(b) withdrawing the liquid reaction composition from the carbonylation reactor and introducing at least part of the withdrawn liquid reaction composition, with or without the addition of heat, to a flash zone to form a vapour fraction comprising, acetic acid product, water, methyl acetate and methyl iodide, and a liquid fraction comprising involatile iridium catalyst, involatile optional promoter or promoters, acetic acid and water,
(c) recycling the liquid fraction from the flash zone to the carbonylation reactor,
(d) introducing the vapour fraction from the flash zone into a distillation zone comprising at least two distillation columns, and
(e) withdrawing from the distillation zone a stream rich in acetic and low in water,
characterised in that the inventory of the liquid reaction composition in the carbonylation reactor is maintained by recycling at least a portion of said acetic acid-rich stream to said carbonylation reactor wherein the acetic acid-rich stream comprises at least 90% by weight of acetic acid.

The present invention provides a means of controlling the inventory of the liquid reaction composition in the reactor by introducing an acid stream into the reactor. The composition in the reactor is practically unaffected by changes in the recycle flow.

By rich in acetic acid and low in water is meant a stream comprising by weight at least 90% acetic acid, preferably at least 95% acetic acid.

The stream rich in acetic acid may typically be any suitable stream withdrawn from one of the distillation columns. Suitably, the base stream from the second (drying) column or the head/side stream from the third (heavy) column may be appropriate streams. In each case, the stream is substantially pure acetic acid. Alternatively, the wet feed stream into the second (drying column) may be used.

The process of the present invention is for the production of acetic acid from methanol and/or a reactive derivative thereof. Suitable reactive derivatives of methanol include methyl acetate, dimethyl ether and methyl iodide. A mixture of methanol and reactive derivatives thereof may be used as reactants in the process of the present invention. Preferably, methanol and/or methyl acetate are used as reactants. If methyl acetate or dimethyl ether are used water co-reactant is also required to produce acetic acid. At least some of the methanol and/or reactive derivative thereof will be converted to, and hence present as, methyl acetate in the liquid reaction composition by reaction with acetic acid product or solvent. The methyl acetate concentration in the liquid reaction composition is suitably in the range from 1 to 70% by weight, preferably from 2 to 50% by weight and more preferably from 5 to 40% by weight.

The carbon monoxide fed to the carbonylation reactor may be essentially pure or may contain inert impurities such as carbon dioxide, methane, hydrogen, nitrogen, noble gases, water and C₁ to C₄ paraffinic hydrocarbons. The partial pressure of carbon monoxide in the carbonylation reactor is suitably in the range from (1 to 70 bar), 10⁵ to 7·10⁶ Pa preferably from 1 to 35 bar, more preferably from (1 to 20 bar) 10⁵ to 2·10⁶ Pa.

The concentration of methyl iodide co-catalyst in the liquid reaction composition is suitably greater than 4% by weight, typically from 4 to 20% by weight, preferably from 4 to 16% by weight. As the methyl iodide concentration in the liquid reaction composition is increased, the amount of propionic acid by-product decreases.

Suitably, the molar ratio of methyl iodide: iridium in the liquid reaction composition is (greater than 20): 1, preferably (up to 400):1, more preferably (from 20 to 200):1. As the molar ratio of methyl iodide: iridium catalyst in the liquid reaction composition is increased, the amount of propionic acid by-product decreases.

The iridium catalyst in the liquid reaction composition may comprise any iridium-containing compound which is soluble in the liquid reaction composition. The iridium catalyst may be added to the liquid reaction composition for the carbonylation reaction in any suitable form which dissolves in the liquid reaction composition or is convertible to a soluble form. Examples of suitable iridium-containing compounds which may be added to the liquid reaction composition include IrCl₃, IrBr₃, [Ir(CO)₂I]₂, [Ir(CO)₂Cl]₂, [Ir(CO)₂Br]₂, [Ir(CO)₂I₂-H⁺, [Ir(CO)₂Br₂]-H⁺, [Ir(CO)₂I₄]-H⁺, [Ir(CH₃)I₃ (CO)₂]-H⁺, IrCl₃.3H₂O, Ir₄(CO)₁₂, iridium metal, Ir₂O₃, IrO₂ Ir(acac)(CO)₂, Ir(acac)₃, iridium acetates, [Ir₃O(Oac)₆(H₂O)₃][Oac], and hexachloroiridic acid [H₂IrCl₂], preferably, chloride-free complexes of iridium such as acetates, oxalates and acetoacetates which are soluble in one or more of the carbonylation reaction components such as water, alcohol and/or carboxylic acid. Particularly preferred is green iridium acetate which may be used in an acetic acid or aqueous acetic acid solution. The concentration of iridium in the liquid reaction composition in the reactor is suitably less than 2500 ppm, preferably from 400 to 2000 ppm.

In the process of the present invention optionally one or more promoters may be present in the reaction composition. Suitable promoters are preferably selected from the group consisting of ruthenium, osmium, rhenium, cadmium, mercury, zinc, gallium, indium and tungsten, and are more preferably selected from ruthenium and osmium and most preferably is ruthenium. Preferably, the promoter is present in an effective amount up to the limit of its solubility in the liquid reaction composition and/or any liquid process streams recycled to the carbonylation reactor from the acetic acid recovery stage. The promoter is suitably present in the liquid composition at a molar ratio of promoter : iridium of from 0.5 : 1 to 15 :1.

The promoter may comprise any suitable promoter metal-containing compound which is soluble in the liquid reaction composition. The promoter may be added to the liquid reaction composition for the carbonylation reaction in any suitable form which dissolves in the liquid reaction composition or is convertible to soluble form. Examples of suitable ruthenium-containing compounds which may be used as sources of promoter include ruthenium (III) chloride, ruthenium (III) chloride trihydrate, ruthenium (IV) chloride, ruthenium (III) bromide, ruthenium metal, ruthenium oxides, ruthenium (III) formate, [Ru(CO)₃I₃]-H⁺, [Ru(CO)₂I₂]ₙ, [Ru(CO)₄I₂], [Ru(CO)₃I₂]₂. tetra(aceto)chlororuthenium (II.III), ruthenium (III) acetate, ruthenium (III) propionate, ruthenium (III) butyrate, ruthenium pentacarbonyl, trifutheniumdodecacarbonyl and mixed ruthenium halocarbonyls such as dichlorotricarbonylruthenium (II) dimer, dibromotricarbonyl-ruthenium (II) dimer, and other organoruthenium complexes such as tetrachlorobis (4-cymene)diruthenium(II), tetra-chlorobis(benzene)diruthenium(II), dichloro(cycloocta- 1,5-diene)ruthenium (II) polymer and tris(acetylacetonate)-ruthenium (III).

Examples of suitable osmium-containing compounds which may be used as sources of promoter include osmium (III) chloride hydrate and anhydrous, osmium metal, osmium tetraoxide, triosmiumdodecacarbonyl, [Os(CO)₄l₂], [Os(CO)₃l₂]₂, [Os(CO)₃l₃]-H⁺, and mixed osmium halocarbonyl such as tricarbonyldichloroosmium (II) dimer and other organoosmium complexes.

Examples of suitable rhenium-containing compounds which may be used as sources of promoter include Re₂(CO)₁₀, Re(CO)₅Cl, Re(CO)₅Br, Re(CO)₅l, ReCl₃.xH₂O [Re(CO)₄l]₂, [Re(CO)₄l₂]-H⁺ and ReCl₅yH₂O.

Examples of suitable cadmium-containing compounds which may be used include Cd(Oac)₂, CdI₂, CdBr₂, CdCl₂ Cd(OH)₂ and cadmium acetylacetonate.

Examples of suitable mercury-containing compounds which may be used as sources of promoter include Hg (Oac)₂, Hgl₂, HgBr₂, HgCl₂, and Hg₂Cl₂. Example of suitable zinc-containing compounds which may be used as sources of promoter include Zn(Oac)₂, Zn(OH)₂, ZnI₂, ZnBr₂, ZnCl₂, and zing acetylacetonate.

Examples of suitable gallium-containing compounds which may be used as sources of promoter include gallium acetylacetonate, gallium acetate, GaCl₃, GaBr₃, Gal₃, Ga₂Cl₄ and Ga(OH)₃.

Examples of suitable indium-containing compounds which may be used as sources of promoter include indium acetylacetonate, indium acetate, InCl₃, InBr₃, InI₃, Inl and In(OH)₃.

Examples of suitable tungsten-containing compounds which may be used as sources of promoter include W(CO)₆, WCl₄, WCl₆, WBr₅, WI₂ or C₉H₁₂ W(CO)₃.

Preferably, the iridium- and promoter-containing compounds are free of impurities which provide or generate in situ ionic iodides which may inhibit the reaction, for example, alkali or alkaline earth metal or other metal salts.

Ionic contaminants such as, for example, (a) corrosion metals, particularly nickel, iron and chromium and (b) phosphines or nitrogen-containing compounds or ligands which may quaternise in situ should be kept to a minimum in the liquid reaction composition as these may generally have an adverse effect on the reaction by generating I- in the liquid reaction composition which may have an adverse effect on the reaction rate. Some corrosion metal contaminants such as for example molybdenum have been found to be less susceptible to the generation of I- Corrosion metals which have an adverse affect on the reaction rate may be minimised by using suitable corrosion resistant materials of construction. Similarly, contaminants such as alkali metal iodides, for example lithium iodide, may be kept to a minimum. Corrosion metal and other ionic impurities may be reduced by the use of a suitable ion exchange resin bed to treat the reaction composition, or preferably a catalyst recycle stream. Such a process is described in US 4007130. Ionic contaminants may be kept below a concentration at which they would generate less than 500 ppm I-, preferably less than 250 ppm I- in the liquid reaction composition.

Water may be formed in situ in the liquid reaction composition, for example, by the esterification reaction between methanol reactant and acetic acid product. Water may be introduced to the carbonylation reactor together with or separately from other components of the liquid reaction composition. Water may be separated from other components of the reaction composition withdrawn from the carbonylation reactor and may be recycled in controlled amounts to maintain the required concentration of the water in the liquid reaction composition. Suitably, the concentration of water in the liquid reaction composition is maintained in the range from 0.5 to 8% by weight.

The carbonylation reactor is suitably maintained at a pressure in the range from (10 to 200 barg) (10⁶ to 2·10⁷) Pag, preferably from (15 to 100 barg) 1,5·10⁶ to 1·10⁷ Pag, more preferably from (15 to 50 barg) 1,5·10⁶ to 5·10⁶ Pag.

The carbonylation reactor is suitably maintained at a temperature in the range from 100 to 300°C, preferably in the range from 150 to 220°C.

The process of the present invention is preferably performed as a continuous process but may be performed as a batch process.

The distillation zone may comprise at least two distillation columns, conventionally three distillation columns. The distillation columns may be referred to as the lights end column, the drying column and the heavy ends column. Where there are two distillation columns, the light ends and drying column may be combined.

When the process is carried out on a plant comprising three distillation columns, in the first distillation column, the methyl iodide and methyl acetate is separated from the acetic and water. Preferably, an acid stream rich in water may be removed at the base of the first distillation zone or at a point one or more stages above the base of the distillation zone. The process stream containing acetic acid may be withdrawn as a liquid or as a vapour. When the process stream is withdrawn as a vapour, preferably a small liquid bleed is also taken from the base of the distillation zone. Suitably, the methyl iodide/methyl acetate stream is removed from the top of the column and recycled back to the reactor.

The wet acetic acid obtained from the first distillation column is withdrawn and passed to a second distillation column to reduce the amount of water therein. A base stream comprising essentially dry acetic acid and little water is passed in whole or part to the heavy ends column. At least part of this stream may be recycled back to the reactor to maintain the inventory of liquid reaction composition in the reactor.

The acid stream from the second column comprises by-products generally referred to as heavy liquid by-product. In order to obtain highly pure acetic acid products, such by-products must be separated. This is done in the heavy ends or third distillation column. The purified acetic acid is removed from the top or the side of the heavy ends (third) distillation column whilst the heavy by-products are removed from the bottom of the column. At least part of the acid product and/or column reflux may be recycled from the heavy ends (third) column to the reactor to maintain the level of liquid reaction composition in the reactor.

In general, the inventory of the liquid reaction composition in the reactor may be maintained by recycling at least part of streams obtained from the second distillation (drying) column feed and/or the second column base stream and/or the purified acetic acid product of the heavy ends column and/or the reflux of the heavy ends column. The inventory of the liquid reaction composition in the reactor may also be maintained by recycling at least part of a combined column (combined light ends and drying column) base stream and/or the purified acetic acid product of the heavy ends column and/or reflux of the heavy ends column.

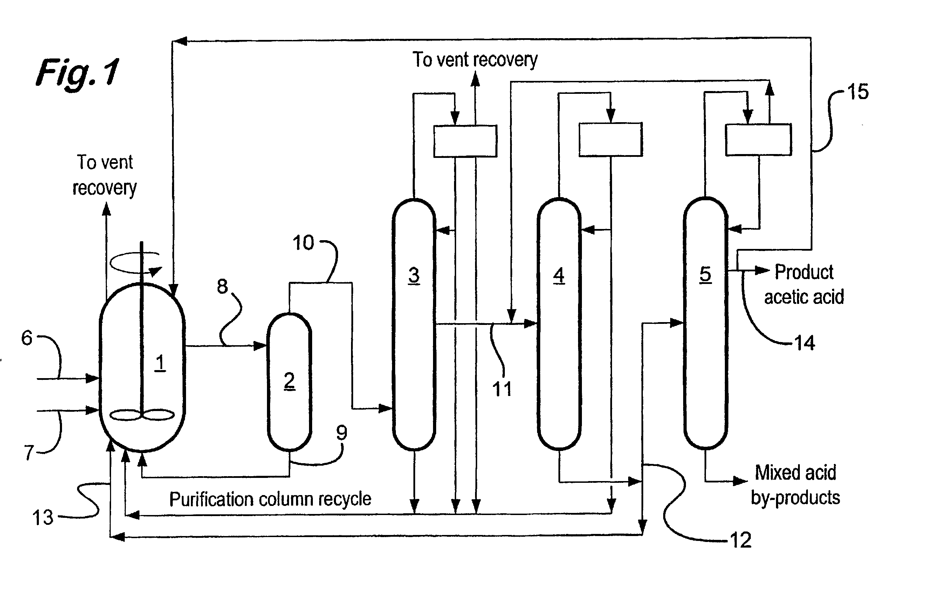

The invention will now be illustrated by reference to the following Figure 1 which represents schematically a plant for the production of acetic aid according to the present invention and comprises a liquid phase carbonylation reactor (1), a flash zone, (2) and a distillation zone comprising a light ends column (3). a drying column (4) and a heavy ends column (5). The carbonylation reactor suitably comprises a stirred reaction vessel within which the reacting liquid components are maintained at a constant level in a liquid reaction composition. Methanol is continuously introduced via line (6), carbon monoxide is fed via line (7).

The liquid reaction composition is withdrawn from the reactor via line (8) and is introduced into the flash zone (2). In the flash zone (2) the liquid components, (catalyst and acetic acid) are withdrawn as a base stream and recycled to the reactor via line 9. The vapour components are withdrawn from the flash zone (2) and passed into the first distillation zone (light ends column) (3) via line (10). Aqueous acetic acid is passed to the second distillation column (4) via line (11). Aqueous methyl iodide and methyl acetate is recycled from the head of the second distillation column to the reactor. In the second distillation column, water is removed from the acetic acid. The dry acetic acid is passed into the third column (5) via line (12). Part of this stream may be recycled back to the reactor to maintain the inventory of the reaction composition in the reactor via line (13). The dry acetic acid and mixed acid by-product are separated in column (5). Acetic acid product is withdrawn via line (14). At least some of this product may be recycled back to the reactor to maintain the inventory of the reaction composition in the reactor via line (15).

## Claims

1. A process for the production of acetic acid which comprises the steps of :
(a) feeding methanol and/or a reactive derivative thereof and carbon monoxide to a carbonylation reactor in which there is maintained during the course of the process a liquid reaction composition comprising an iridium catalyst, methyl iodide co-catalyst, a finite amount of water, methyl acetate and optionally one or more promoters,
(b) withdrawing the liquid reaction composition from the carbonylation reactor and introducing at least part of the withdrawn liquid reaction composition, with or without the addition of heat, to a flash zone to form a vapour fraction comprising, acetic acid product, water, methyl acetate and methyl iodide, and a liquid fraction comprising involatile iridium catalyst, involatile optional promoter or promoters, acetic acid and water,
(c) recycling the liquid fraction from the flash zone to the carbonylation reactor,
(d) introducing the vapour fraction from the flash zone into a distillation zone comprising at least two distillation columns, and
(e) withdrawing from the distillation zone a stream rich in acetic and low in water,
**characterised in that** the inventory of the reaction composition in the carbonylation reactor is maintained by recycling at least a portion of said acetic acid-rich stream to said carbonylation reactor,
and wherein the acetic acid rich stream comprises at least 90% by weight of acetic acid.

2. A process as claimed in claim 1 wherein the acetic acid-rich stream comprises as least 95% by weight acetic acid.

3. A process as claimed in claim 1 or claim 2 wherein the distillation zone comprises three distillation columns in which (i) an acid stream rich in water, is removed at the base of the first distillation zone or at a point one or more stages above the base of the first distillation zone and fed to a second distillation column to reduce the amount of water therein, (ii) a base stream comprising essentially dry acetic acid, little water and heavy by-products is passed from the second distillation column to a heavy ends column and (iii) purified acetic acid is removed from the top or the side of the heavy ends distillation column whilst the heavy by-products are removed from the bottom of the column.

4. A process as claimed in claim 3 wherein the inventory of the liquid reaction composition in the reactor is maintained by recycling at least part of streams obtained from the second distillation column feed and/or the second column base stream and/or the purified acetic acid product of the heavy ends column and/or reflux of the heavy ends column.

5. A process as claimed in claim 3 modified in that the first and second columns are combined and the level of the liquid reaction composition in the reactor is maintained by recycling at least part of the combined column base stream and/or the purified acetic acid product of the heavy ends column and/or reflux of the heavy ends column.

## Patentansprüche

1. Verfahren zur Herstellung von Essigsäure, das die Schritte umfasst:
(a) Zuführen von Methanol und/oder einem reaktiven Derivat davon und Kohlenmonoxid zu einem Carbonylierungsreaktor, in dem im Lauf des Verfahrens eine flüssige Reaktionszusammensetzung, die einen Iridiumkatalysator, Methyljodidcokatalysator, eine endliche Menge Wasser, Essigsäuremethylester und gegebenenfalls einen oder mehrere Promotoren umfasst, aufrechtgehalten wird,
(b) Abziehen der flüssigen Reaktionszusammensetzung aus dem Carbonylierungsreaktor und Einführen von mindestens einem Teil der abgezogenen flüssigen Reaktionszusammensetzung, mit oder ohne Zusatz von Wärme, zu der Entspannungszone unter Erzeugung einer Dampffraktion, umfassend Essigsäureprodukt, Wasser, Essigsäuremethylester und Methyljodid und einer flüssigen Fraktion, umfassend nicht flüchtigen Iridiumkatalysator, nicht flüchtigen gegebenenfalls vorliegenden Promotor oder Promotoren, Essigsäure und Wasser,
(c) Zurückführen der flüssigen Fraktion aus der Entspannungszone zu dem Carbonylierungsreaktor,
(d) Einführen der Dampffraktion aus der Entspannungszone in eine mindestens zwei Destillationskolonnen umfassende Destillationszone und
(e) Abziehen aus der Destillationszone eines an Essigsäure reichen und an Wasser armen Stroms,
**dadurch gekennzeichnet, dass** die Bestandteile der Reaktionszusammensetzung in dem Carbonylierungsreaktor durch Zurückführen von mindestens einem Teil des an Essigsäure reichen Stroms zu dem Carbonylierungsreaktor aufrechtgehalten wird und worin der an Essigsäure reiche Strom mindestens 90 Gew.-% Essigsäure umfasst.

2. Verfahren nach Anspruch 1, wobei der an Essigsäure reiche Strom mindestens 95 Gew.-% Essigsäure umfasst.

3. Verfahren nach Anspruch 1 oder Anspruch 2, wobei die Destillationszone drei Destillationskolonnen umfasst, worin (i) ein an Säure reicher Strom in Wasser an der Basis der ersten Destillationszone oder an einem Punkt einen oder mehrere Böden oberhalb der Basis der ersten Destillationszone entfernt wird und einer zweiten Destillationskolonne zugeführt wird, um die Wassermenge darin zu vermindern, (ii) ein Basisstrom, umfassend im Wesentlichen trockene Essigsäure, etwas Wasser und hoch siedende Nebenprodukte aus der zweiten Destillationskolonne zu einer Kolonne für hoch siedende Stoffe geleitet wird, und (iii) gereinigte Essigsäure von dem Oberen oder Seite der Destillationskolonne für hoch siedende Stoffe entfernt wird, während die hoch siedenden Nebenprodukte vom Sumpf der Kolonne entfernt werden.

4. Verfahren nach Anspruch 3, wobei die Bestandteile der flüssigen Reaktionszusammensetzung in dem Reaktor durch Zurückführen von mindestens einem Teil der aus der zweiten Destillationskolonnenzuführung und/oder dem zweiten Kolonnenbasisstrom und/oder dem gereinigten Essigsäureprodukt der Kolonne für hoch siedende Stoffe und/oder von dem Rückfluss der Kolonne für hoch siedende Stoffe erhaltenen Ströme aufrechterhalten wird.

5. Verfahren nach Anspruch 3, dadurch modifiziert, dass die ersten und zweiten Kolonnen kombiniert werden und der Anteil der flüssigen Reaktionszusammensetzung in dem Reaktor durch Zurückführen von mindestens einem Teil des kombinierten Kolonnenbasisstroms und/oder des gereinigten Essigsäureprodukts der Kolonne für hoch siedende Stoffe und/oder Rückfluss der Kolonne für hoch siedende Stoffe aufrechterhalten wird.

## Revendications

1. Procédé pour la production d'acide acétique, qui comprend les étapes consistant :
(a) à introduire du méthanol et/ou un de ses dérivés réactifs et du monoxyde de carbone dans un réacteur de carbonylation dans lequel est maintenue au cours de la mise en oeuvre du procédé une composition réactionnelle liquide comprenant un catalyseur à l'iridium, un cocatalyseur consistant en iodure de méthyle, une quantité finie d'eau, de l'acétate de méthyle et facultativement un ou plusieurs promoteurs,
(b) à décharger la composition réactionnelle liquide du réacteur de carbonylation et à introduire au moins une partie de la composition réactionnelle liquide déchargée, avec ou sans chauffage, dans une zone de détente pour former une fraction de vapeur comprenant le produit consistant en acide acétique, de l'eau, de l'acétate de méthyle et de l'iodure de méthyle, et une fraction liquide comprenant le catalyseur à l'iridium non volatile, le ou les promoteurs facultatifs non volatiles, de l'acide acétique et de l'eau,
(c) à recycler la fraction liquide de la zone de détente au réacteur de carbonylation,
(d) à introduire la fraction de vapeur provenant de la zone de détente dans une zone de distillation comprenant au moins deux colonnes de distillation, et
(e) à décharger de la zone de distillation un courant riche en acide acétique et pauvre en eau,
**caractérisé en ce que** le stock de la composition réactionnelle dans le réacteur de carbonylation est maintenu en recyclant au moins une partie dudit courant riche en acide acétique audit réacteur de carbonylation, le courant riche en acide acétique comprenant au moins 90 % en poids d'acide acétique.

2. Procédé suivant la revendication 1, dans lequel le courant riche en acide acétique comprend au moins 95 % en poids d'acide acétique.

3. Procédé suivant la revendication 1 ou la revendication 2, dans lequel la zone de distillation comprend trois colonnes de distillation dans lesquelles (i) un courant acide riche en eau est évacué à la base de la première zone de distillation ou à un point situé à un ou plusieurs étages au-dessus de la base de la première zone de distillation et est amené à une deuxième colonne de distillation pour réduire la quantité d'eau qui s'y trouve, (ii) un courant de base comprenant essentiellement de l'acide acétique anhydre, une petite quantité d'eau et des sous-produits lourds est passé de la deuxième colonne de distillation à une colonne de fractions lourdes, et (iii) l'acide acétique purifié est évacué par la partie supérieure ou la partie latérale de la colonne de distillation des fractions lourdes, tandis que les sous-produits lourds sont évacués par le fond de la colonne.

4. Procédé suivant la revendication 3, dans lequel le stock de la composition liquide réactionnelle dans le réacteur est maintenu en recyclant au moins une partie des courants obtenus à partir de la charge d'alimentation de la deuxième colonne de distillation et/ou du courant de base de la deuxième colonne et/ou du produit consistant en acide acétique purifié de la colonne de fractions lourdes et/ou du reflux de la colonne de fractions lourdes.

5. Procédé suivant la revendication 3, modifié en combinant les première et deuxième colonnes et en maintenant la quantité de la composition réactionnelle liquide dans le réacteur en recyclant au moins une partie du courant de base des colonnes combinées et/ou du produit consistant en acide acétique purifié de la colonne de fractions lourdes et/ou du reflux de la colonne de fractions lourdes.
